# EUROPÄISCHE PATENTSCHRIFT

(11) **EP 1 881 857 B1**
(45) Veröffentlichungstag und Bekanntmachung des Hinweises auf die Patenterteilung: **17.04.2013**
(21) Anmeldenummer: 06753631.8
(22) Anmeldetag: 15.05.2006
(51) Int. Cl.: A61M 1/36

(54) **VERFAHREN ZUM LUFTFREIEN FÜLLEN DER BLUTSEITE EINER HÄMODIALYSEVORRICHTUNG MIT EINER PHYSIOLOGISCHEN ELEKTROLYTLÖSUNG**
METHOD FOR THE AIR BUBBLE-FREE FILLING OF THE BLOOD-CONTAINING END OF A HEMODIALYZER WITH A PHYSIOLOGICAL ELECTROLYTE SOLUTION
PROCEDE POUR REMPLIR, SANS AIR, LE COTE SANG D'UN DISPOSITIF D'HEMODIALYSE AU MOYEN D'UNE SOLUTION ELECTROLYTIQUE PHYSIOLOGIQUE

(30) Priorität: 17.05.2005 DE 102005022545
(43) Veröffentlichungstag der Anmeldung: 30.01.2008
(73) Patentinhaber: Fresenius Medical Care Deutschland GmbH, 61352 Bad Homburg v.d.H. (DE)
(72) Erfinder: APEL, Jörn, 22927 Grosshansdorf (DE); FISCHER, Max, 60323 Frankfurt (DE)
(74) Vertreter: Laufhütte, Dieter
(86) Internationale Anmeldenummer: PCT/EP2006/004565
(87) Internationale Veröffentlichungsnummer: WO 2006/122737

(56) Entgegenhaltungen:
- EP-A- 0 161 686
- DE-C1- 10 011 208
- US-A- 5 228 889
- US-A1- 2002 007 137

## Beschreibung

Die Erfindung betrifft ein Verfahren zum luftfreien Befüllen der Blutseite einer Hämodialysevorrichtung mit Flüssigkeit wie einer physiologischen Elektrolytlösung und zum Entlüften der Blutseite einer Hämodialysevorrichtung während des Betriebes.

Es ist bereits bekannt, zur Abscheidung von Luft in extrakorporalen Blutkreisläufen im blutabführenden Leitungssystem, also im sogenannten venösen Teil des extrakorporalen Blutkreislaufs als Kammer ausgebildete Luftabscheider vorzusehen. In diesen Luftabscheidem stellt sich gewöhnlich während der Benutzung ein Luftkissen über einem Flüssigkeitspegel ein, da eine permanente Luftverbindung zwischen dem Inneren der Kammer und der Umgebung besteht. Es wird auch diskutiert, ob ein derartiger Blut-Luftkontakt aus Blutkompatibilitätsgründen vermieden werden sollte. Daher ist bereits in der US 5,849,065 A ein Luftabscheider vorgeschlagen worden, der vollständig mit Blut gefüllt ist. Ein derartiger Luftabscheider hat aber den Nachteil, dass eine hydrophobe Membran, die den Austritt von Blut verhindern soll, ständig in Kontakt mit dem Blut selbst steht. Dies kann nun zum Verstopfen der Membran führen, und darüber hinaus steht das Blut selbst über die Membranoberfläche weiterhin in Kontakt mit der Luft, da die Membran ja eine Porosität von ca. 60% aufweist und somit nur optisch eine Luftbarriere darstellt. In den verwendeten Ausführungsformen werden speziell beschichtete Luftabscheidermembranen, die für Blutsysteme entwickelt wurden, verwendet. Diese speziell entwickelten Membranen sind vergleichsweise teuer. Bei einer Beschädigung der Membran ergibt sich ein vergleichsweise hohes Sicherheitsrisiko, da hier zum einen Blut austreten kann und zum anderen die Gefahr einer Kreuzkontamination besteht.

Das Befüllen des extrakorporalen Blutkreislaufs von Dialysemaschinen wird beispielhaft in der EP 0 560 368 A2 sowie der DE 100 11 208 C1 beschrieben.

Aus der US 2003/0,135,152 A ist das nachträgliche Abscheiden von Luft aus einem System bekannt. Die US 2002/0017489 A erläutert den Einsatz einer Abscheideleitung mit einer Klemme und einem Rückschlagventil.

Aus der EP 0 161 686 B1 ist es bereits bekannt ein Ventil vorzusehen und hinter dem Ventil eine herkömmliche hydrophobe Membran anzuordnen.

Aufgabe der vorliegenden Erfindung ist es, ein Verfahren zur luftfreien Befüllung mit Flüssigkeit, insbesondere physiologischer Elektrolytlösung an die Hand zu geben.

Erfindungsgemäß wird diese Aufgabe durch ein einfaches und kostengünstiges Verfahren mit den Merkmalen des Anspruchs 1 gelöst. Hierzu wird beim Befüllen das Leitungssystem einschließlich einer in diesem angeordneten Kammer mit Flüssigkeit, insbesondere physiologischer Elektrolytlösung befüllt, wobei ein oberhalb der Kammer angeordnetes Ventil geöffnet wird, um das System zu entlüften. Das Ventil wird wieder geschlossen, wenn die Flüssigkeit ein oberhalb des Ventils angeordnetes Hydrophobfilter oder die Leitung oberhalb des Ventils erreicht hat, das heißt das Ventil wird geschlossen, sobald sich keine Luft mehr unterhalb des Ventils befindet. Nach Schließen des Ventils ist sichergestellt, dass das gesamte System vollkommen luftfrei ist und dass hier auch kein Kontakt mehr zur Luft besteht. Die Grenzfläche zwischen der eingefüllten Kochsalzlösung und der Luft befindet sich außerhalb des abgetrennten Bereichs, das heißt oberhalb des Ventils. Während des sich anschließenden Betriebs kann das Blut nur noch mit der in der Leitung verbleibenden Elektrolytlösungssäule in Verbindung stehen. Aufgrund einer Volumenminimierung der Leitungssysteme kann die Trennfläche zwischen dem Blut und der Elektrolytlösungssäule zusätzlich minimiert werden. Somit wird eine Berührung von Blut mit einem entsprechenden Hydrophobfilter vermieden. Dadurch bedarf es auch nicht des Einsatzes der teueren und aufwendig hergestellten Hydrophobfilter, die mit dem Blut in Kontakt treten.

Vorteilhafte Ausgestaltungen der Erfindung ergeben sich aus den sich an den Hauptanspruch anschließenden Unteransprüchen.

So kann entsprechend Anspruch 2 während des Betriebes angesammelte Luft durch Öffnen des Ventils aufgrund bsp. nachströmenden Blutes aus der Kammer verdrängt werden. Das entsprechende sich in der Kammer ansammelnde Luftvolumen kann mit Hilfe eines Niveausensors detektiert und durch kurzzeitiges Öffnen des Ventils wieder entfernt werden. Dabei trifft eventuell nachströmendes Blut auf die zuvor erwähnte Elektrolytlösungssäule beziehungsweise -schicht.

Das vollständige Entweichen der Luft während des Füllens mit der physiologischen Elektrolytlösung erfolgt vorteilhaft über die Messung des Druckverlaufs im blutabführenden Leitungssystem, da bei entsprechendem Druckanstieg darauf geschlossen werden kann, dass die physiologische Elektrolytlösung die Membran berührt. Bei Detektieren dieses Druckanstiegs kann der Füllvorgang mit der physiologischen Elektrolytlösung beendet werden. Die Steuerung der Entlüftung kann auch über eine vorgegebene Flußsteuerung der in die Kammer fördernden Pumpe durchgeführt werden.

Die Erfindung betrifft nach Anspruch 7 weiterhin eine Blutbehandlungsvorrichtung zur Durchführung des Verfahrens nach Anspruch 1. Diese Blutbehandlungsvorrichtung weist eine Steuereinheit auf, mittels der das Verfahren durchgeführt werden kann.

Weitere Merkmale, Einzelheiten und Vorteile der Erfindung ergeben sich aus einem Ausführungsbeispiel, das anhand der beigefügten Zeichnung näher erläutert wird. Es zeigen:
- Figur 1:: eine schematisch dargestellte Ausführungsform der Erfindung und
- Figur 2:: ein Detail der Ausführungsform gemäß Figur 1.

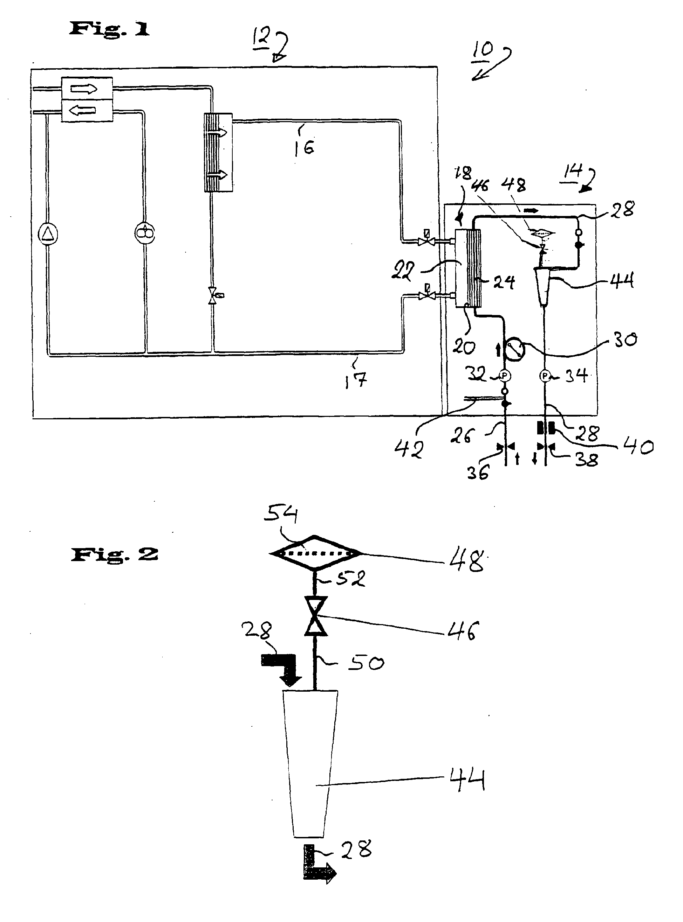

In der Figur 1 ist ein erfindungsgemäßes Hämodialysegerät 10 dargestellt. Ein solches Gerät weist in an sich bekannter Weise einen Dialysierflüssigskeitskreislauf 12 und einen extrakorporalen Blutkreislauf 14 auf.

Der hier dargestellte Dialysierflüssigkeitskreislauf ist konventionell aufgebaut. Die Dialysierflüssigkeit gelangt von einer nicht näher dargestellten Dialysierflüssigkeitsquelle über die Dialysierflüssigkeitszuführleitung 16 zum Hämodialysator 18. Der Hämodialysator 18 ist durch eine semipermeable Membran 20 in eine Dialysierflüssigkeitskammer 22 und eine Blutkammer 24 unterteilt. Die Dialysierflüssigkeitskammer 22 des Hämodialysators 18 ist einerseits mit der Dialysierflüssigkeitszuführleitung 16 und andererseits mit der Dialysierflüssigkeitsabführleitung 17 verbunden.

Bezüglich des weiteren Aufbaus des Dialysierflüssigkeitskreislaufs 12, der in Figur 1 nur schematisch gezeigt ist, kann beispielsweise auf die Beschreibung der DE 100 11 208 A1 verwiesen werden. Es kann hier auch ein beliebig anderer Aufbau gewählt werden.

Der extrakorporale Blutkreislauf 14 weist folgenden Aufbau auf. Die Blutkammer 24 weist eine Zuführleitung 26 und eine Abführleitung 28 auf. Der Volumenstrom wird durch ein Pumpmittel 30 eingestellt. In den jeweiligen Leitungen 26 und 28 sind Drucksensoren 32 bzw. 34 angeordnet. Die Zuführleitung 26 kann über eine Klemme 36 abgeklemmt werden, während die Abführleitung 28 durch eine Klemme 38 abgeklemmt werden kann. In der Leitung 28 ist zusätzlich eine Blasendetektoreinheit 40 vorgesehen. Mit 42 ist ein Anschluss in der Zuführleitung 26 für eine Antikuagulanzmittelinfusion (z. B. Heparin) gezeigt.

Im senkrecht angeordneten Teil der Abführleitung 28 ist eine Kammer 44 vorgesehen, an deren oberen Bereich die von der Blutkammer 24 kommende Abführleitung einmündet und in deren unteren Bereich der weiterführende Teil der abführenden Leitung 28 angesetzt ist. Beim Betrieb des Hämodialysegerätes 10 füllt sich die Kammer 44 auf. Idealer Weise ist die Kammer 44 vollständig mit Flüssigkeit, das heißt bei Betrieb des Hämodialysegeräts mit Blut, gefüllt. Im oberen Bereich der Kammer ist ein in der Figur 1 nur schematisch dargestelltes Ventil 46 angeordnet. Oberhalb des Ventils wiederum ist ein Hydrophobfilter 48 vorgesehen. Der genauere Aufbau der Kammer 44 mit Ventil 46 und Hydrophobfilter 48 ergibt sich aus der Figur 2. Dort ist der einmündende Teil der Abführleitung 28 sowie der abführende Teil der Abführleitung 28 im Bereich der Kammer 44 gezeigt. Vom oberen Teil der Kammer 44 zweigt, wie in dem Ausführungsbeispiel gemäß Figur 2 dargestellt wurde, eine Leitung, beispielsweise Schlauchleitung oder Rohrleitung oder ein sonstiger Kanal 50 ab, mit welchem das Ventil 46 verbunden ist. Das Ventil kann auch direkt an der Kammerbegrenzung angeordnet sein. Das Ventil 46 ist wiederum über eine Schlauchleitung, Rohrleitung oder einem sonstigen Kanal 52 mit dem oberhalb des Ventils angeordneten Hydrophobfilter 48 verbunden.

Vor Inbetriebnahme des Hämodialysegeräts 10 wird der extrakorporale Blutkreislauf 14 mit einer physiologischen Elektrolytlösung, in der Regel einer physiologischen Kochsalzlösung gefüllt. Erst anschließend wird der Patient angeschlossen und das entsprechende Blut durch den extrakorporalen Blutkreislauf mittels des Pumpmittels 30 gepumpt. Hierbei wird dann die physiologische Elektrolytlösung durch das nachströmende Blut verdrängt.

Gemäß der vorliegenden Erfindung wird zum luftfreien Füllen der Blutseite des Hämodialysegeräts 10 beim Befüllen das blutabführende Leitungssystem 28 einschließlich der Kammer 44 mit physiologischer Elektrolytlösung gefüllt, wobei das Ventil 46 geöffnet bleibt, bis die physiologische Elektrolytlösung bis an die Membran 54 des Hydrophobfilters 48 gelangt ist oder zumindest die Leitung oberhalb des Ventils erreicht hat. Anschließend wird das Ventil 46 geschlossen, so dass in dem Bereich oberhalb der Kammer 44, nämlich in dem Verbindungskanal 50 bzw. 52 eine physiologische Elektrolytlösung steht. Sollte das Ventil 46 direkt an der Kammerbegrenzung angeordnet sein, so verbleibt die physiologische Kochsalzlösung zumindest in dem Verbindungskanal 52.

Bei Beginn der Dialyse wird die physiologische Elektrolytlösung aus den Leitungen 26 der Blutkammer 24, der Leitung 28 und der Kammer 44 verdrängt. Sollte sich nun während der Dialyse Luft im oberen Kammerbereich 44 ansammeln, was durch eine hier nicht näher dargestellte Füllstandsmessung in der Kammer 44 detektiert werden kann, kann zum Entlüften das Ventil 46 geöffnet werden, so dass die Luft durch die Leitung 50 bzw. 52 über das Hydrophobfilter 48 entweichen kann. In diesem Fall kann eventuell Blut in die Leitung 50 nachströmen, wobei hier jedoch kein Kontakt mit der Außenatmosphäre erfolgt, sondern nur ein Kontakt über den Querschnitt der Leitung 50 mit der physiologischen Elektrolytlösung, die in der Leitung steht.

Der Befüllvorgang während des Befüllens des extrakorporalen Kreislaufs 14 mit der physiologischen Elektrolytlösung kann über die Drucksensoren 32 und 34 gesteuert werden, da nach Erreichen der Membran 54 im Hydrophobfilter 48 der Systemdruck ansteigt. Bei Feststellen dieses Druckanstiegs wird das zunächst geöffnete Ventil 46 geschlossen. Das Ventil 46 kann ein maschinenseitig ansteuerbares Schlauchklemmventil sein, wenn die Leitung 50 bzw. 52 als Schlauch ausgeführt ist. Als Hydrophobfilter 48 läßt sich ein kostengünstiger herkömmlicher Hydrophobfilter einsetzen. Alternativ kann die in die Kammer fördernde Pumpe, z. B. die Blutpumpe, so betrieben werden, dass sie ein vorgegebenes Volumen an Flüssigkeit in die Kammer fördert, so dass sich keine Luft mehr unterhalb des Ventils befindet.

In der Ausführungsform, bei der der Abgang der Kammer 44 zum Ventil 46 bei geschlossener Stellung einen toten und schmalen Zweig darstellt, nutzt die Erfindung zusätzlich die Tatsache, dass der Zweig aufgrund der Kapillarkräfte vollständig mit Kochsalzlösung gefüllt ist und somit weder verklottet noch zu Luftkontakt führt.

Selbst eine undichte oder fehlende Membran führt bei der erfindungsgemäßen Vorrichtung nicht zu einem Sicherheitsrisiko, da allenfalls Kochsalzlösung beim Füllen in die Umgebung gelangen kann. Dies wird aber auch bei einem automatischen Befüllen durch die Maschine dadurch bemerkbar, dass kein Druckanstieg in der Kammer beim Füllvorgang detektiert wird.

## Patentansprüche

1. Verfahren zum luftfreien Füllen der Blutseite einer Hämodialysevorrichtung mit einer Flüssigkeit, insbesondere einer physiologischen Kochsalzlösung,
**dadurch gekennzeichnet,**
**daß** beim Füllen das Leitungssystems einschließlich einer in diesem angeordneten Kammer (44) mit Flüssigkeit, gefüllt wird, wobei ein oberhalb der Kammer (44) angeordnetes Ventil (46) geöffnet wird, um die Kammer (44) zu entlüften und wobei das Ventil (46) wieder geschlossen wird, wenn die Flüssigkeit ein oberhalb des Ventils (46) angeordnetes Hydrophobfilter (48) oder zumindest die Leitung oberhalb des Ventils (46) erreicht hat, so dass sich keine Luft mehr unterhalb des Ventils (46) befindet.

2. Verfahren nach Anspruch 1, **dadurch gekennzeichnet, daß** zum Entlüften einer Hämodialysevorrichtung während des Betriebes die während des Betriebes angesammelte Luft durch Öffnen des Ventils (46) aufgrund nachströmenden Blutes aus der Kammer (44) verdrängt wird.

3. Verfahren nach Anspruch 1 oder 2, **dadurch gekennzeichnet, daß** das vollständige Entweichen der Luft während des Befüllens über die Messung des Druckverlaufs im blutführenden Leitungssystem oder dem damit in Fluidverbindung stehenden Dialysierflüssigkeits-Kreislauf gemessen wird.

4. Verfahren nach Anspruch 1 oder 2, **dadurch gekennzeichnet, daß** das vollständige Entweichen der Luft während des Befüllens über eine Messung des Füllstandes in der Leitung oberhalb des Ventils (46) erfolgt.

5. Verfahren nach Anspruch 1 oder 2, **dadurch gekennzeichnet, daß** das vollständige Entweichen der Luft durch die Detektion des Füllstandes mittels eines an die Kammer angekoppelten Niveausensors an einer Stelle unterhalb des Ventils (46) und einem anschließenden, volumenkontrollierten Füllen des Restvolumens der Kammer (44) mittels der in die Kammer (44) fördernden Pumpe erfolgt.

6. Verfahren nach Anspruch 5, **dadurch gekennzeichnet, dass** die Pumpe die Blutpumpe der Hämodialysevorrichtung ist.

7. Verfahren nach Anspruch 1 oder 2, **dadurch gekennzeichnet, dass** das Füllen des Kreislaufs dadurch beendet wird, dass ein beliebiger Füllstand im zu füllenden Leitungssystem detektiert wird und danach die zum Füllen benutzte Pumpe eine vordefinierte, von der Förderrate abhängige Zeit weiterfördert.

8. Verfahren nach Anspruch 1, 2, 3 und/oder 4, **dadurch gekennzeichnet, daß** der Füllstand der Kammer (44) während des Betriebes überwacht wird.

9. Verfahren nach Anspruch 8, **dadurch gekennzeichnet daß** bei Feststellen einer Luftansammlung während des Betriebes das Ventil (46) geöffnet wird, bis über einen Füllstandsanzeiger oberhalb des Ventils (46), eine Messung des Druckverlaufs oder einer volumenkontrollierten Füllung der Kammer (44) mittels der in die Kammer (44) fördernden Pumpe das vollständige Entweichen der Luft erreicht wurde.

10. Blutbehandlungsvorrichtung zur Durchführung eines der Verfahren nach Anspruch 1 bis 9, mit einem extrakorporalen Blutkreislauf und einer in dieser angeordneten Kammer (44), mit einem oberhalb der Kammer (44) abzweigenden Kanal (50) und einem in diesem Kanal (50) angeordneten Hydrophobfilter (48), mit einem in dem Kanal (50) angeordneten Ventil (46), und mit einer Fördereinrichtung von Flüssigkeit in dem extrakorporalen Blutkreislauf,
**dadurch gekennzeichnet,**
**dass** die Vorrichtung ferner eine Steuereinheit aufweist, die so konfiguriert ist, dass sie den extrakorporalen Kreislauf durch Betrieb der Fördereinrichtung mit Flüssigkeit befüllen kann und das Ventil in Abhängigkeit der Meßwerte einer den Füllstand in dem Kanal (50) erfassenden Sensors oder nach einer vorgegebenen volumenkontrollierten Füllung der Kammer (44) schließt, um einen Füllstand der Flüssigkeit im Kanal zwischen dem Ventil und der Hydrophobfiltermembran zu erreichen.

11. Blutbehandlungsvorrichtung nach Anspruch 10, **dadurch gekennzeichnet, dass** der Sensor ein Drucksensor zur Erfassung des Drucks in der Kammer (44) oder des Drucks in einem mit der Kammer (44) in Verbindung stehendem Fluidsystem ist.

12. Blutbehandlungsvorrichtung nach Anspruch 10, **dadurch gekennzeichnet, dass** der Sensor ein an der Leitung zwischen dem Ventil (46) und der Hydrophobfiltermembran (48) gelegener Niveausensor ist.

13. Blutbehandlungsvorrichtung nach Anspruch 10, **dadurch gekennzeichnet, dass** zur volumenkontrollierten Füllung mit Hilfe eines Sensors ein beliebiger Füllstand im zu füllenden Leitungssystem detektiert wird und die Steuereinheit nach Detektion dieses Füllstands die Fördereinrichtung mit einer vordefinierten, von der der Förderrate abhängigen Zeit betreibt.

14. Blutbehandlungsvorrichtung nach Anspruch 13, **dadurch gekennzeichnet, dass** der Sensor ein an die Kammer (44) angekoppelter Niveausensor ist.

15. Blutbehandlungsvorrichtung nach einem der Ansprüche 10 bis 14, **dadurch gekennzeichnet, dass** die Steuereinheit zum Entlüften der Hämodialysevorrichtung während des Betriebs die während des Betriebs angesammelte Luft durch Öffnen des Ventils (46) aufgrund nachströmenden Blutes aus der Kammer (44) verdrängt.

16. Blutbehandlungsvorrichtung nach einem der Ansprüche 10 bis 15, **dadurch gekennzeichnet, dass** ein Ventil in der von der Kammer abführenden Leitung des extrakorporalen Kreislaufs während des Befüll- oder Entlüftungsvorgangs von der Steuereinheit geschlossen wird.

## Claims

1. A method for the air-free filling of the blood side of a hemodialysis apparatus with a liquid, in particular with a physiological saline solution,
**characterized in that**,
during filling, the line system, including a chamber (44) arranged therein, is filled with liquid, with a valve (46) arranged above the chamber (44) being opened to vent the chamber (44), and with the valve (46) being closed again when the liquid has reached a hydrophobic filter (48) arranged above the valve (46), or at least the line above the valve (46), so that air is no longer located beneath the valve (46).

2. A method in accordance with claim 1, **characterized in that** the air collected during operation is displaced from the chamber (44) by opening the valve (46) due to inflowing blood for the venting of a hemodialysis apparatus during operation.

3. A method in accordance with either of claims 1 or 2, **characterized in that** the complete escape of the air during filling is measured via the measurement of the pressure development in the blood-conducting line system or in the dialysis liquid circuit in fluid communication therewith.

4. A method in accordance with either of claims 1 or 2, **characterized in that** the complete escape of the air during filling takes place via a measurement of the filling level in the line above the valve (46).

5. A method in accordance with either of claims 1 or 2, **characterized in that** the complete escape of the air takes place by the detection of the filling level by means of a level sensor coupled to the chamber at a position beneath the valve (46) and a subsequent volume-controlled filling of the residual volume of the chamber (44) by means of the pump pumping into the chamber (44).

6. A method in accordance with claim 5, **characterized in that** the pump is the blood pump of the hemodialysis apparatus.

7. A method in accordance with either of claims 1 or 2, **characterized in that** the filling of the circuit is terminated **in that** any desired filling level in the line system to be filled is detected and then the pump used for filling continues to pump for a pre-defined time dependent on the delivery rate.

8. A method in accordance with claim 1, claim 2, claim 3 and/or claim 4, **characterized in that** the filling level of the chamber (44) is monitored during operation.

9. A method in accordance with claim 8, **characterized in that**, when an air collection is determined during operation, the valve (46) is opened until the complete escape of the air has been reached, via a filling level indicator above the valve (46), via a measurement of the pressure development or of a volume-controlled filling of the chamber (44) by means of the pump pumping into the chamber (44).

10. A blood treatment apparatus for the carrying out of one of the methods in accordance with claims 1 to 9, having an extracorporeal blood circuit and a chamber (44) arranged therein, having a passage (50) branching off above the chamber (44) and a hydrophobic filter (48) arranged in this passage (50), having a valve (46) arranged in the passage (50) and having a conveying unit of liquid in the extracorporeal blood circuit,
**characterized in that**
the apparatus furthermore has a control unit configured such that it can fill the extracorporeal circuit with liquid by operation of the conveying unit and the valve closes in dependence on the measured values of a sensor detecting the filling level in the passage (50) or in accordance with a predetermined volume-controlled filling of the chamber (44) to achieve a filling level of the liquid in the passage between the valve and the hydrophobic filter membrane.

11. A blood treatment apparatus in accordance with claim 10, **characterized in that** the sensor is a pressure sensor for the detection of the pressure in the chamber (44) or of the pressure in a fluid system in communication with the chamber (44).

12. A blood treatment apparatus in accordance with claim 10, **characterized in that** the sensor is a level sensor disposed at the line between the valve (46) and the hydrophobic filter membrane (48).

13. A blood treatment apparatus in accordance with claim 10, **characterized in that** any desired filling level is detected in the line system to be filled for the volume-controlled filling with the help of a sensor and the control unit operates the conveying unit after detection of this filling level with a predetermined time dependent on the pumping rate.

14. A blood treatment apparatus in accordance with claim 13, **characterized in that** the sensor is a level sensor coupled to the chamber (44).

15. A blood treatment apparatus in accordance with one of the claims 10 to 14, **characterized in that** the control unit displaces the air collected during operation for the venting of the hemodialysis apparatus on the basis of inflowing blood from the chamber (44) by opening the valve (46).

16. A blood treatment apparatus in accordance with any one of the claims 10 to 15, **characterized in that** a valve in the line of the extracorporeal circuit leading away from the chamber is closed by the control unit during the filling or venting process.

## Revendications

1. Procédé destiné au chargement sans air du côté sang d'un dispositif d'hémodialyse avec un liquide, notamment une solution de chlorure de sodium physiologique,
**caractérisé en ce que**,
lors du chargement, le système de conduits comprenant une chambre disposée dans celui-là (44) est chargé en liquide, une vanne (46) disposée en amont de la chambre (44) étant ouverte pour purger la chambre (44) et la vanne (46) étant refermée lorsque le liquide a atteint un filtre hydrophobe (48) disposé en amont de la vanne (46) ou au moins le conduit au-dessus de la vanne (46), de sorte qu'il n'y ait plus d'air à se trouver en aval de la vanne (46).

2. Procédé selon la revendication 1, **caractérisé en ce que**, pour la purge du dispositif d'hémodialyse pendant le fonctionnement, l'air accumulé pendant le fonctionnement est refoulé par l'ouverture de la vanne (46) en raison du sang affluant ultérieurement à partir de la chambre (44).

3. Procédé selon les revendications 1 ou 2, **caractérisé en ce que** le dégagement complet de l'air pendant le chargement est mesuré via la mesure de la courbe de pression dans le système de conduits conducteur de sang ou le circuit de liquide de dialyse se trouvant en relation fluide avec celui-ci.

4. Procédé selon les revendications 1 ou 2, **caractérisé en ce que** le dégagement complet de l'air pendant le chargement s'effectue via une mesure du niveau de remplissage dans le conduit en amont de la vanne (46).

5. Procédé selon les revendications 1 ou 2, **caractérisé en ce que** le dégagement complet de l'air s'effectue par la détection du niveau de remplissage au moyen d'un capteur de niveau accouplé à la chambre à un endroit en aval de la vanne (46) et sur un chargement à volume contrôlé ultérieur du volume restant de la chambre (44) au moyen de la pompe transporteuse dans la chambre (44).

6. Procédé selon la revendication 5, **caractérisé en ce que** la pompe est la pompe à sang du dispositif d'hémodialyse.

7. Procédé selon les revendications 1 ou 2, **caractérisé en ce que** le chargement du circuit est terminé **en ce qu'**un niveau de chargement quelconque est détecté dans le système de conduits à charger et la pompe utilisée pour le chargement poursuit ensuite le transport pendant une période prédéfinie dépendante du taux transporté.

8. Procédé selon les revendications 1, 2, 3 et/ou 4, **caractérisé en ce que** le niveau de remplissage de la chambre (44) est surveillé pendant le fonctionnement.

9. Procédé selon la revendication 8, **caractérisé en ce que**, en cas de constatation d'une accumulation d'air pendant le fonctionnement, la vanne (46) est ouverte, jusqu'à ce que, par l'intermédiaire d'un afficheur de niveau de remplissage en amont de la vanne (46), par une mesure de la courbe de pression ou d'un chargement à volume contrôlé de la chambre (44), le dégagement complet de l'air soit atteint au moyen de la pompe de transport dans la chambre (44).

10. Dispositif de traitement sanguin destiné à l'exécution d'un des procédés selon les revendications 1 à 9, avec un circuit sanguin extracorporel et une chambre (44) disposée dans celui-ci, avec un canal (50) dérivant en amont de la chambre (44) et un filtre hydrophobe (48) disposé dans ledit canal (50) avec une vanne (46) disposée dans le canal (50) et avec un dispositif de transport de liquide dans le circuit sanguin extracorporel,
**caractérisé en ce que**
le dispositif présente par ailleurs une unité de commande qui est configurée de sorte qu'elle peut remplir de liquide le circuit extracorporel par le fonctionnement du dispositif de transport et **en ce que** la vanne se ferme en fonction des valeurs de mesure d'un capteur saisissant le niveau de remplissage dans le canal (50) ou après un chargement à volume contrôlé de la chambre afin d'atteindre un niveau de remplissage dans le canal entre la vanne et la membrane de filtre hydrophobe.

11. Dispositif de traitement sanguin selon la revendication 10, **caractérisé en ce que** le capteur est un capteur de pression destiné à la saisie de la pression dans la chambre (44) ou de la pression dans un système fluide se trouvant en relation avec la chambre (44).

12. Dispositif de traitement sanguin selon la revendication 10, **caractérisé en ce que** le capteur est un capteur de niveau placé sur le conduit entre la vanne (46) et la membrane de filtre hydrophobe (48).

13. Dispositif de traitement sanguin selon la revendication 10, **caractérisé en ce que**, pour le chargement à volume contrôlé à l'aide d'un capteur, un niveau de remplissage quelconque est détecté dans le système de conduits à remplir et **en ce que** l'unité de commande actionne le dispositif de transport après la détection dudit niveau de remplissage avec un temps prédéfini dépendant du taux de transport.

14. Dispositif de traitement sanguin selon la revendication 13, **caractérisé en ce que** le capteur est un capteur de niveau accouplé à la chambre (44).

15. Dispositif de traitement sanguin selon une quelconque des revendications 10 à 14, **caractérisé en ce que** l'unité de commande destinée à la purge du dispositif d'hémodialyse pendant le fonctionnement refoule l'air accumulé pendant le fonctionnement par l'ouverture de la vanne (46) en raison du sang affluant hors de la chambre (44).

16. Dispositif de traitement sanguin selon une quelconque des revendications 10 à 14, **caractérisé en ce qu'**une vanne est fermée par l'unité de commande dans le conduit d'évacuation hors de la chambre du circuit extracorporel pendant l'opération de chargement ou de purge.
